# EUROPEAN PATENT APPLICATION

(11) **EP 2 196 221 A1**
(43) Date of publication of application: **16.06.2010**
(21) Application number: 08834316.5
(22) Date of filing: 26.09.2008
(51) Int. Cl.: A61K 47/10, A61K 9/20, A61K 47/26, A61K 47/38

(54) **RAPIDLY DISINTEGRATING SOLID PREPARATION**

(30) Priority: 27.09.2007 JP 2007250920
(71) Applicant: Mitsubishi Tanabe Pharma Corporation, Osaka-shi Osaka 541-8505 (JP)
(72) Inventor: OOKAWA, Akiko, Osaka-shi Osaka 541-8505 (JP); IMADA, Yasushi, Osaka 541-8505 (JP); SHINKAI, Yasunari, Osaka-shi Osaka 541-8505 (JP); MATSUMOTO, Hideaki, Osaka-shi Osaka 541-8505 (JP)
(74) Representative: von Kreisler Selting Werner
(86) International application number: PCT/JP2008/067549
(87) International publication number: WO 2009/041651

(57) **Abstract**

Provided is a solid preparation which rapidly disintegrates in the presence of saliva or a small amount of water in the oral cavity, particularly, a rapidly disintegrating solid preparation useful as an orally-disintegrating solid preparation.
Specifically provided is a rapidly disintegrating solid preparation containing coated granules wherein saccharide or sugar alcohol having an average particle size of not less than 75 µm and a high dissolution rate is coated with cellulose, and a rapidly disintegrating solid preparation containing a) an active ingredient, b) saccharide or sugar alcohol having an average particle size of not less than 400 µm, c) cellulose and d) a disintegrant.

## Description

### Technical Field

The present invention relates to a solid preparation which rapidly disintegrates in the presence of saliva or a small amount of water in the oral cavity, particularly, a rapidly disintegrating solid preparation useful as an orally-disintegrating solid preparation.

### Background Art

With the advent of an aging society, a preparation having a form easily taken by elderly people is desired. As the situation stands, however, most of the oral preparations are general tablets and capsules, which are not necessarily easy to take for elderly people. Moreover, such general preparations are often difficult to take for children and patients having difficulty in swallowing. In addition, powder and granule are problematic in handling after opening, adhesion in the oral cavity and the like, and are not satisfactory for elderly people, children and patients having difficulty in swallowing. To solve such problems, some preparations have already been tried with regard to tablets that can be taken even without water and are easily handleable.

WO97/47287 (patent document 1) describes that an orally rapidly disintegrating tablet superior in disintegration property and hardness can be obtained by compression molding a mixture of sugar alcohol or saccharide having an average particle size of 30 µm or below, an active ingredient and a disintegrant.

JP-A-2001-58944 (patent document 2) describes a rapidly disintegrating solid preparation containing an active ingredient, D-mannitol having an average particle size of 30 µm - 300 µm, a disintegrant and cellulose. It discloses that an orally disintegrating tablet having a hardness free of practical problems even at a low dry compression pressure, and having rapid disintegration property and free of problems in producibility can be obtained by mixing an active ingredient, a comparatively crude saccharide or sugar alcohol, a disintegrant and cellulose at once and compression molding the mixture.

JP-A-2007-197438 (patent document 3) discloses an orally disintegrating tablet using spherical granules containing D-mannitol having a bulk density of not less than about 0.6 g/mL. It discloses an orally disintegrating tablet using D-mannitol substantially having an average particle size of 200 µm - 250 µm and a bulk density of about 0.72 g/mL.

W02004/064810 (patent document 4) discloses an orally disintegrating tablet composed of coated granules wherein a core composed of granules containing a medicament or a core composed of granules containing a medicament and saccharide is coated with a disintegrant.

JP-A-2004-315483 (patent document 5) discloses an orally disintegrating tablet composed of granules obtained by granulating saccharide or sugar alcohol using a water-insoluble, hydrophilic granulation component. According to patent document 5, rapid disintegration is achieved by coating the surface of granules with a water-insoluble, hydrophilic granulation component (e.g., starch, silicic acid etc.).
However, none of these patent documents describe or suggest that a compact orally rapidly disintegrating solid preparation having superior disintegration time as well as sufficient hardness can be produced efficiently by using, as a component of the preparation, coated granules wherein saccharide or sugar alcohol having a particular average particle size and a particular dissolution rate is coated with cellulose.

patent document 1: WO97/47287
patent document 2: JP-A-2001-58944
patent document 3: JP-A-2007-197438
patent document 4: WO2004/064810
patent document 5: JP-A-2004-315483

### Disclosure of the Invention

### Problems to be Solved by the Invention

Under such circumstances, convenient and economical production of a tablet further having sufficient hardness that does not prevent smooth handling from tablet molding to ingestion thereof, comfortable during use and superior in disintegration property in the oral cavity has been desired.

### Means of Solving the Problems

The present inventors have conducted intensive studies of convenient and economical production of an orally rapidly disintegrating solid preparation superior in rapid disintegration property and economic aspect without using a special component, and found that a superior and compact orally rapidly disintegrating solid preparation having sufficient hardness and a disintegration time of within 30 seconds, preferably within 15 seconds, can be produced efficiently by using coated granules wherein saccharide or sugar alcohol having an average particle size of not less than 75 µm and a high dissolution rate is coated with cellulose, which resulted in the completion of the present invention.
Moreover, the present inventors conducted intensive studies of convenient and economical production of an orally rapidly disintegrating solid preparation superior in rapid disintegration property and economic aspect without using a special component, and found that a superior and compact orally rapidly disintegrating solid preparation having sufficient hardness and a disintegration time of within 15 seconds can be produced efficiently by mixing saccharide or sugar alcohol having an average particle size of not less than 400 µm, cellulose and a disintegrant, granulating them such that other additive is present on the surface of saccharide or sugar alcohol by using equipment such as a centrifugal rotary granulating-coating apparatus or a twin-screw kneader and the like, and compression molding them, which resulted in the completion of the present invention.

According to the present invention, a superior and compact orally rapidly disintegrating solid preparation having sufficient hardness and a disintegration time of within 30 seconds, preferably within 15 seconds, can be produced efficiently by using coated granules wherein saccharide or sugar alcohol having an average particle size of not less than 75 µm and a high dissolution rate is coated with cellulose. Accordingly, the present invention provides the following.
(1) A rapidly disintegrating solid preparation comprising coated granules wherein saccharide or sugar alcohol having an average particle size of not less than 75 µm and a high dissolution rate is coated with cellulose.
(2) The solid preparation of the above-mentioned (1), wherein said saccharide or sugar alcohol is a crystal particle or a granulated particle.
(3) The solid preparation of the above-mentioned (2), wherein said saccharide or sugar alcohol is a crystal particle.
(4) The solid preparation of any of the above-mentioned (1) to (3), wherein said saccharide or sugar alcohol has an average particle size of not less than 100 µm.
(5) The solid preparation of the above-mentioned (4), wherein said saccharide or sugar alcohol has an average particle size of 100 µm - 700 µm.
(6) The solid preparation of the above-mentioned (5), wherein said saccharide or sugar alcohol has an average particle size of 100 µm - 400 µm.
(7) The solid preparation of any of the above-mentioned (1) to (5), wherein said saccharide or sugar alcohol has an average particle size of not less than 400 µm.
(8) The solid preparation of any of the above-mentioned (1) to (7), wherein said saccharide or sugar alcohol has a dissolution rate of not less than 4.0 [1/min·m²].
(9) The solid preparation of the above-mentioned (8), wherein said saccharide or sugar alcohol has a dissolution rate of not less than 4.5 [1/min·m²].
(10) The solid preparation of any of the above-mentioned (1) to (9), wherein said saccharide or sugar alcohol is one or more kinds selected from erythritol, α-form mannitol and lactose.
(11) The solid preparation of the above-mentioned (10), wherein said saccharide or sugar alcohol is erythritol.
(12) The solid preparation of any of the above-mentioned (1) to (11), having a hardness of not less than 35N and a disintegration time of 30 seconds or less.
(13) The solid preparation of the above-mentioned (12), wherein said hardness is not less than 35N and said disintegration time is 15 seconds or less.
(14) The solid preparation of any of the above-mentioned (1) to (13), wherein said cellulose for coating is 8 - 30 parts by weight relative to 100 parts by weight of said saccharide or sugar alcohol.
(15) The solid preparation of the above-mentioned (14), wherein said cellulose for coating is 10 - 20 parts by weight relative to 100 parts by weight of said saccharide or sugar alcohol.
   According to the present invention, moreover, the stable tabletability and tablet property necessary for general handling can be achieved while maintaining rapid disintegration property in the oral cavity, by using saccharide or sugar alcohol having an average particle size of not less than 400 µm and comprising an additive on the surface thereof. Accordingly, the present invention provides the following.
(16) A rapidly disintegrating solid preparation comprising a) an active ingredient, b) saccharide or sugar alcohol having an average particle size of not less than 400 µm, c) cellulose and d) a disintegrant.
(17) The preparation of the above-mentioned (16), which is an orally rapidly disintegrating solid preparation.
(18) The preparation of the above-mentioned (16), comprising a compression molded product of granules comprising the active ingredient, and at least one kind of additive from c) cellulose and d) a disintegrant present on the surface of said saccharide or sugar alcohol having an average particle size of not less than 400 µm.
(19) The preparation of the above-mentioned (16), wherein said saccharide or sugar alcohol is contained in 40 - 90 parts by weight relative to 100 parts by weight of said preparation.
(20) The preparation of the above-mentioned (16), wherein said saccharide is lactose, sucrose or trehalose.
(21) The preparation of the above-mentioned (16), wherein said sugar alcohol is one or more kinds selected from D-mannitol, erythritol, xylitol, sorbitol and maltitol.
(22) A method of producing the preparation of the above-mentioned (16), comprising compression molding a mixture comprising a) an active ingredient, b) saccharide or sugar alcohol having an average particle size of not less than 400 µm, c) cellulose and d) a disintegrant.

### Effect of the Invention

As mentioned above, a superior and compact orally rapidly disintegrating solid preparation having sufficient hardness, for example, not less than 35N, and a disintegration time of within 30 seconds, preferably within 15 seconds, can be produced efficiently by using coated granules wherein saccharide or sugar alcohol having an average particle size of not less than 75 µm and a high dissolution rate is coated with cellulose.
As mentioned above, moreover, since an additive (cellulose, disintegrant) is present on the surface of saccharide or sugar alcohol having an average particle size of not less than 400 µm, segregation during tableting can be prevented, and a rapidly disintegrating solid preparation having a hardness of the same level as general tablets and a disintegration time in the oral cavity of 15 seconds or less can be obtained.

### Brief Description of the Drawings

Fig. 1 shows the state of the surface of granules obtained by powder coating granulation of erythritol having an average particle size of 430 µm obtained by milling and screening. (Example 7)
Fig. 2 shows the state of the surface of a physical mixture of erythritol having an average particle size of 430 µm obtained by milling and screening. (Comparative Example 4)

### Best Mode for Carrying out the Invention

The drug to be used in the present invention (sometimes to be also referred to as "active ingredient") may be any of solid, crystal, oil, solution and the like and, for example, one or more kinds of components selected from nutritional supplement, antipyretic analgesic antiphlogistic drug, psychotropic drug, antianxiety drug, antidepressant, sedative-hypnotic drug, antispasmolytic drug, central nervous system drug, brain metabolic stimulant, brain circulation improver, antiepilepsy agent, sympathomimetic drug, gastrointestinal drug, digestive drug, antacid, antiulcerogenic agent, antitussive expectorant, antiemetic, anapnoic, bronchodilator, antiallergic drug, dental and oral drug, antihistamine agent, cardiotonic agent, antiarrhythmic agent, diuretic, antihypertensive agent, vasoconstrictor, coronary vasodilator, peripheral vasodilator, hypolipidemic agent, cholagogue, antibiotic, chemotherapeutic agent, diabetic agent, drug for osteoporosis, anti-rheumatic drug, skeleton muscle relaxant, spasmolytic, hormone agent, narcotic alkaloid, sulfa drug, therapeutic drug for gout, intravascular coagulation inhibitor, antimalignant tumor agent and the like are used.

As the nutritional supplement, arginine, taurine and the like can be mentioned. As the antipyretic analgesic antiphlogistic drug, acetaminophen, ibuprofen, aspirin and the like can be mentioned. As the psychotropic drug, bromperidol, cariprazine hydrochloride, risperidone, olanzapine, methylphenidate, barbital and the like can be mentioned. As the antianxiety drug, etizolam, flutazolam, fludiazepam and the like can be mentioned. As the antidepressant, nortriptyline hydrochloride, trazodone hydrochloride and the like can be mentioned. As the sedative-hypnotic drug, triazolam, flurazepam and the like can be mentioned. As the antispasmolitic drug, atropine hydrochloride, piperidolate hydrochloride, tiquizium bromide and the like can be mentioned. As the central nervous system drug, clocapramine hydrochloride, olanzapine and the like can be mentioned. As the brain metabolic stimulant, aniracetam, ifenprodil tartrate and the like can be mentioned. As the brain circulation improver, nicergoline, ibudilast and the like can be mentioned. As the antiepilepsy agent, sodium valproate, phenytoin, clorazepate dipotassium and the like can be mentioned. As the sympathomimetic drug, ephedrine and the like can be mentioned. As the gastrointestinal drug, sulpiride, lansoprazole and the like can be mentioned. As the digestive drug, sofalcone, famotidine and the like can be mentioned. As the antacid, sodium hydrogen carbonate, magnesium silicate and the like can be mentioned. As the antiulcerogenic agent, sucralfate, polaprezinc and the like can be mentioned.

As the antitussive expectorant, potassium guaiacolsulfonate, guaifenesin and the like can be mentioned. As the antiemetic, hydroxyzine pamoate, ramosetron hydrochloride, granisetron sulfate and the like can be mentioned. As the anapnoic, naloxone hydrochloride, flumazenil and the like can be mentioned. As the bronchodilator, procaterol hydrochloride, theophylline and the like can be mentioned. As the antiallergic drug, azelastine hydrochloride, Rizaben and the like can be mentioned. As the dental and oral drug, dexamethasone, azulene sulfonate sodium and the like can be mentioned. As the antihistamine agent, diphenhydramine hydrochloride, triprolidine hydrochloride and the like can be mentioned. As the cardiotonic agent, digoxin, deslanoside and the like can be mentioned. As the antiarrhythmic agent, mexiletine hydrochloride, procainamide, amiodarone hydrochloride and the like can be mentioned. As the diuretic, furosemide, acetazolamide and the like can be mentioned. As the antihypertensive agent, metoprolol tartrate, losartan potassium, amlodipine besylate and the like can be mentioned. As the vasoconstrictor, etilefrine hydrochloride, epinephrine and the like can be mentioned. As the coronary vasodilator, nifedipine, dipyridamole and the like can be mentioned. As the peripheral vasodilator, amyl nitrite, papaverine hydrochloride and the like can be mentioned. As the hypolipidemic agent, pravastatin, colestyramine and the like can be mentioned. As the cholagogue, ursodeoxycholic acid, anethole trithione, trepibutone and the like can be mentioned. As the antibiotic, penicillin, cephalosporin, streptomycin and the like can be mentioned. As the chemotherapeutic agent, sulfamethoxazole, tosufloxacin tosilate, hexamine mandelate and the like can be mentioned. As the diabetic agent, pioglitazone hydrochloride, chlorpropamide and the like can be mentioned. As the drug for osteoporosis, ipriflavone, alendronate sodium hydrate and the like can be mentioned. As the anti-rheumatic drug, actarit, salazosulfapyridine and the like can be mentioned. As the skeleton muscle relaxant, chlorzoxazone, chlorphenesin carbamate and the like can be mentioned. As the spasmolytic, butylscopolamine bromide and the like can be mentioned. As the hormone agent, dexamethasone, methyltestosterone and the like can be mentioned. As the narcotic alkaloid, opium, cocaine and the like can be mentioned. As the sulfa drug, sulfamonomethoxine, sulfadimethoxine, sulfanilamide and the like can be mentioned. As the therapeutic drug for gout, colchicine, sulfinpyrazone and the like can be mentioned. As the intravascular coagulation inhibitor, warfarin potassium, enoxaparin sodium and the like can be mentioned. As the antimalignant tumor agent, imatinib mesylate, nelarabine, etoposide and the like can be mentioned.

The amount of the active ingredient to be blended is generally 0.01 wt% - 70 wt%, preferably 0.1 wt% - 50 wt%, more preferably 1 wt% - 10 wt%. The average particle size of the active ingredient is generally not more than 500 µm, preferably 0.1 µm - 100 µm, more preferably 1 µm - 20 µm.

The terms used in the present invention are now explained in the following. In the present specification, a simple indication of wt% means wt% relative to the total amount of one tablet when the total weight of one tablet is 100 wt%.

In the present invention, as the "saccharide and sugar alcohol", any can be used as long as it has an average particle size of not less than 75 µm, and a high dissolution rate.
Here, the "average particle size" generally refers to an average particle size of crystal particles of saccharide or sugar alcohol. In the present invention, saccharide or sugar alcohol crystal particles having an average particle size of 75 µm or below are granulated by a general granulation method, and granulated particles having an average particle size of not less than 75 µm are also included.
Here, a method of granulating saccharide or sugar alcohol to have an average particle size of not less than 75 µm may be any as long as it is used for granulation of general preparations. Usually, a fluid bed granulator, a high shear granulator, an extrusion granulating machine, a rotary fluidized bed granulator, a Wurster fluid bed granulator, or a combination of these granulating machines can also be used.
In the present invention, as the "saccharide", lactose, sucrose, trehalose and the like can be mentioned, with preference given to lactose.
In the present invention, moreover, as the "sugar alcohol", D-mannitol, erythritol, xylitol, sorbitol, maltitol and the like can be mentioned, with preference given to erythritol.
In the present invention, the "average particle size" is measured by, for example, a laser diffraction particle size analyzer [Mastersizer] of Malvern Instruments Ltd. The average particle size of saccharide and sugar alcohol to be used in the present invention is not less than 75 µm, and selected from not less than 100 µm, 100 - 700 µm, 100 - 400 µm and not less than 400 µm.
In the present invention, the "dissolution rate" is obtained by using a 6-vessel dissolution tester (NTR-6100, Toyama Sangyo Co., Ltd.). The paddle rotation speed was 50 min⁻¹, and saccharide or sugar alcohol was added by 2 g to desalted water (500 mL), which is a test solution, heated to 37°C. Samples were taken at given time intervals after addition, changes in the dry weight of the samples per unit time were calculated, and dissolution rate constant k was calculated by the Noyes-Whitney equation. The dissolution rate constant k was converted to value per surface area of each saccharide or sugar alcohol and the obtained value was taken as dissolution rate [1/min·m²].
As the specific surface area, the value measured by a laser diffraction particle size analyzer [Mastersizer] of Malvern Instruments Ltd. was used.
In the present invention, saccharide and sugar alcohol having a high dissolution rate particularly have a dissolution rate of not less than 4.0 [1/min·m²], preferably, not less than 4.5 [1/min·m²], as measured by the above-mentioned method.
As the saccharide having an average particle size of not less than 400 µm, lactose, sucrose, trehalose and the like can be mentioned. As the sugar alcohol having an average particle size of not less than 400 µm, D-mannitol, erythritol, xylitol, sorbitol, maltitol and the like can be mentioned, with preference given to erythritol. In addition, saccharide and sugar alcohol, preferably sugar alcohol, more preferably erythritol, to be used in the present invention have an average particle size of not less than 400 µm, and 400 µm - 700 µm saccharide or sugar alcohol is preferable.
The average particle size of saccharide or sugar alcohol to be used in the present invention is measured by, for example, a laser diffraction particle size analyzer [Mastersizer] of Malvern Instruments Ltd.
The amount of saccharide or sugar alcohol to be used in the present invention is 40 - 95 parts by weight, preferably 50 - 90 parts by weight, relative to 100 parts by weight of the solid pharmaceutical preparation.

In the present invention, as the cellulose for coating saccharide or sugar alcohol, low-substituted hydroxypropylcellulose, microcrystalline cellulose, powder cellulose, carmellose (e.g., carmellose calcium, croscarmellose sodium and the like) and the like can be mentioned.
The amount of cellulose to be used in the present invention is 0.5 - 40 parts by weight, preferably 1 - 20 parts by weight, relative to 100 parts by weight of the solid pharmaceutical preparation, and 8 - 30 parts by weight, preferably 10 - 20 parts by weight, relative to 100 parts by weight of said saccharide or sugar alcohol.
In the present invention, the method of coating saccharide or sugar alcohol with cellulose may be any as long as it can cover saccharide or sugar alcohol. Therefore, generally, a fluid bed granulator, a high shear granulator, an extrusion granulator, a rotary fluidized bed granulator, a Wurster fluid bed granulator or a combination of these granulating machines can be used. Coating by a centrifugal rotary granulating-coating apparatus or a twin-screw kneader is preferable.

In the present invention, moreover, as long as the effect of the invention is not impaired, a disintegrant can also be further added along with cellulose. As the disintegrant, carmellose calcium, croscarmellose sodium, sodium carboxymethyl starch, crospovidone and the like can be used. The amount thereof to be used is 0.5 - 15 parts by weight, preferably 1 - 10 parts by weight, relative to 100 parts by weight of the solid pharmaceutical preparation.

In addition, one or more kinds of the active ingredient, cellulose and the disintegrant may be used in combination.

Furthermore, as long as the effect of the invention is not impaired, the preparation of the present invention may contain an adequate amount of starches such as corn starch, potato starch, wheat starch, rice starch, partly pregelatinized starch, porous starch and the like as filler or various additives used for the production of general preparations. As such additive, for example, filler, binder, souring agent, bubbling agent, artificial sweetener, flavor, lubricant, colorant, stabilizer, pH adjuster, surfactant and the like can be mentioned.

As the filler, an inorganic excipient includes anhydrous calcium phosphate, precipitated calcium carbonate, calcium silicate, light anhydrous silicic acid and the like can be mentioned.
As the binder, for example, hydroxypropylcellulose, hydroxypropylmethylcellulose, polyvinylpyrrolidone, gum arabic powder, gelatin, pullulan and the like can be mentioned.

As the souring agent, for example, citric acid, tartaric acid, malic acid, ascorbic acid and the like can be mentioned.

As the bubbling agent, for example, sodium hydrogen carbonate, sodium carbonate and the like can be mentioned. As the sweetener, saccharine sodium, dipotassium glycyrrhizate, aspartame, stevia, thaumatin and the like can be mentioned.

As the flavor, various fruit flavor containing strawberry, yogurt flavor, lemon oil, orange oil, menthol and the like can be mentioned.

As the Lubricant, for example, magnesium stearate, calcium stearate, sucrose fatty acid ester, polyethylene glycol, talc, stearic acid, sodium stearyl fumarate and the like can be mentioned.

As the colorant, for example, food colors such as Food Color Yellow No. 5, Food Color Red No. 2, Food Color Blue No. 2 and the like, food lake colors, red ferric oxide and the like can be mentioned.

As the stabilizer, disodium edetate, tocopherol, cyclodextrin and the like can be mentioned.

As the pH adjuster, citrate, phosphate, carbonate, tartrate, fumarate, acetate, amino acid salt and the like can be mentioned.

As the surfactant, sodium lauryl sulfate, polysorbate 80, hydrogenated oil, polyoxyethylene(160)polyoxypropylene(30)glycol and the like can be mentioned.

While the particle size of each component other than saccharide or sugar alcohol used for the orally rapidly disintegrating solid preparation of the present invention is not particularly limited, a particle size of not more than 500 µm that does not easily cause a gritty texture in the oral cavity is preferable. In addition, any one kind of these components may be used or two or more kinds thereof may be used in combination.

During production of the solid preparation of the present invention, moreover, fine granular core coated with an active ingredient, an additive and the like, and further coated by a known method for the purpose of masking of taste and odor, enteric processing or sustained release and the like may be mixed therewith and used.

The production method of the preparation of the present invention is explained in more detail in the following.

The production of the orally rapidly disintegrating solid preparation of the present invention is not particularly limited as long as it is a general compression molding method.
For example, the coated granules of the present invention wherein saccharide or sugar alcohol having an average particle size of not less than 75 µm and a high dissolution rate is coated with cellulose are produced, after which the coated granules are added with an active ingredient, a disintegrant, and other components generally used for formulation of preparations are appropriately used according to a general formulation method of preparation to give the orally rapidly disintegrating solid preparation of the present invention. For molding of the orally rapidly disintegrating solid preparation of the present invention, the molding method is not particularly limited as long as it is a general compression molding method. A general tableting machine, for example, a rotary tableting machine, an oil hydraulic press machine, a single punch tableting machine and the like can be used.
The pressure for tablet molding is basically the same as that for general tablet production, and is appropriately determined according to the components to be blended.
The solid preparation of the present invention can be produced by, for example, processing a) an active ingredient, b) saccharide or sugar alcohol having an average particle size of not less than 400 µm, c) cellulose and d) a disintegrant into coated granules using equipment such as a centrifugal rotary granulating-coating apparatus, a twin-screw kneader and the like, mixing/spraying a lubricant therewith/thereon, and compression molding the mixture. Alternatively, it can also be produced by processing a) an active ingredient, b) saccharide or sugar alcohol having an average particle size of not less than 400 µm, c) cellulose and/or d) a disintegrant into coated granules using equipment such as a centrifugal rotary granulating-coating apparatus, a twin-screw kneader and the like, adding c) cellulose and/or d) a disintegrant, and compression molding the mixture. As a result, the surface of saccharide or sugar alcohol is coated with additives such as cellulose, a disintegrant and the like, together with the active ingredient.
While a centrifugal rotary granulating-coating apparatus and a twin-screw kneader are recited as the equipment used for coating the surface of saccharide or sugar alcohol with other additives, the equipment is not limited thereto.

Since the thus-obtained rapidly disintegrating solid preparation of the present invention comprises coated granules wherein saccharide or sugar alcohol having an average particle size of not less than 75 µm and a high dissolution rate is coated with cellulose, it can prevent segregation during tableting, and shows easy handleability of the same level as general tablets and a disintegration time in the oral cavity of 30 seconds or less, preferably 15 seconds or less.
In addition, since an additive (cellulose and/or disintegrant) is present on the surface of saccharide or sugar alcohol having an average particle size of not less than 400 µm, it can prevent segregation during tableting, and shows easy handleability of the same level as general tablets and a disintegration time in the oral cavity of 15 seconds or less.

### Examples

The present invention is explained in more detail in the following by referring to Examples and the like, which are not to be construed as limitative.

The tablets obtained in the Examples and Comparative Examples were measured for hardness, disintegration time in the oral cavity, disintegration time (ODT) by the following test methods. In addition, the dissolution rate of saccharide and sugar alcohol used in the Examples and Comparative Examples was measured.
(1) The hardness was measured by a tablet hardness tester (WHT-2ME, Pharma Test). The test was performed using 3 or 10 tablets, and the average value is shown.
(2) As for the disintegration time in the oral cavity, the tablet of the present invention was placed in the oral cavity of a healthy adult without water, and the time necessary for complete disintegration of the tablet with only saliva was measured.
(3) The disintegration time (ODT) was measured by an Orally Disintegrating Tablet Tester (ODT-101, Toyama Sangyo Co., Ltd.). As for the measurement conditions, a weight (15 g, weight diameter 15 mm) was used at a rotating speed of 50 rpm, and an average of 3 measures was taken.
(4) The "dissolution rate" was measured by using a 6-vessel dissolution tester (NTR-6100, Toyama Sangyo Co., Ltd.). The paddle rotation speed was 50 min⁻¹, and saccharide or sugar alcohol was added by 2 g to desalted water (500 mL), which is a test solution, heated to 37°C. Samples were taken at given time intervals after addition, changes in the dry weight of the samples per unit time were calculated, and dissolution rate constant k was calculated by the Noyes-Whitney equation. The dissolution rate constant k was converted to value per the surface area of each saccharide or sugar alcohol and the obtained value was taken as dissolution rate [1/min·m²].
   As the specific surface area, the value measured by a laser diffraction particle size analyzer [Mastersizer] of Malvern Instruments Ltd. was used.

### Example 1

Erythritol (Mitsubishi-Kagaku Foods Corporation, average particle size 610 µm, dissolution rate 11.99 [1/min·m²], 9.4 g), and low-substituted hydroxypropylcellulose (Shin-Etsu Chemical Co., Ltd., 0.6 g) were charged in a twin-screw kneader (Kurimoto, Ltd., S1KRC kneader) and processed at a kneading temperature of 115°C to give kneaded granules.
Microcrystalline cellulose (Asahi Kasei Corporation, 100 mg), croscarmellose sodium (FMC, 100 mg), and light anhydrous silicic acid (Y.K.F. Inc., 17 mg) were added to the obtained granules (1.8 g) and they were mixed. The mixture was tableted (KIKUSUI SEISAKUSHO LTD., correct 12HUK, tablet diameter 8 mmΦ flat plane corner angle, compression pressure 1000 kgf) to give 200 mg tablets.

### Example 2

Erythritol (Mitsubishi-Kagaku Foods Corporation, average particle size 610 µm, 9.4 g), and microcrystalline cellulose (0.6 g) were charged in a twin-screw kneader and processed at a kneading temperature of 115°C to give kneaded granules. Low-substituted hydroxypropylcellulose (100 mg), croscarmellose sodium (100 mg), and light anhydrous silicic acid (,17 mg) were added to the obtained granules (1.8 g) and they were mixed. The mixture was tableted (tablet diameter 8 mmΦ flat plane corner angle, compression pressure 1000 kgf) to give 200 mg tablets.

### Example 3

Erythritol (Mitsubishi-Kagaku Foods Corporation, average particle size 610 µm, 500 g) was charged in a centrifugal rotary granulating-coating apparatus (Freund Corporation, GX-20), and a mixture of microcrystalline cellulose (30 g), low-substituted hydroxypropylcellulose (30 g) and croscarmellose sodium (30 g) was added while spraying purified water (76 g), and the mixture was subjected to powder coating granulation, which was followed by a drying step to give granules. Light anhydrous silicic acid (5.2 g) was added to the obtained granules (542 g) and they were mixed. Magnesium stearate was sprayed (external lubricating method) and the mixture was tableted (tablet diameter 7 mmΦ flat plane corner angle, compression pressure 1000 kgf, 1200 kgf) to give 150 mg tablets.

### Example 4

Erythritol (Mitsubishi-Kagaku Foods Corporation, average particle size 610 µm, 500 g) was charged in a centrifugal rotary granulating-coating apparatus, and low-substituted hydroxypropylcellulose (30 g) was added while spraying purified water (83 g), and the mixture was subjected to powder coating granulation, which was followed by a drying step to give granules. Microcrystalline cellulose (30 g), croscarmellose sodium (30 g), and light anhydrous silicic acid (5.3 g) were added to the obtained granules (506 g) and they were mixed. Magnesium stearate was sprayed (external lubricating method) and the mixture was tableted (tablet diameter 7 mmΦ flat plane corner angle, compression pressure 1000 kgf) to give 150 mg tablets.

### Example 5

Erythritol (Mitsubishi-Kagaku Foods Corporation, average particle size 610 µm, 850 g), microcrystalline cellulose (50 g), low-substituted hydroxypropylcellulose (50 g), and croscarmellose sodium (50 g) were charged in a twin-screw kneader and processed at a kneading temperature of 109°C to give kneaded granules. The obtained granules were subjected to milling and screening in a screen mill (POWREX CORPORATION, COMIL, screen hole diameter 2 mm grater-type). Light anhydrous silicic acid (4.6 g) was added to the obtained screened granules (542 g) and they were mixed. Magnesium stearate was sprayed (external lubricating method) and the mixture was tableted (tablet diameter 7 mmΦ flat plane corner angle, compression pressure 1000 kgf) to give 150 mg tablets.

### Example 6

Erythritol (Mitsubishi-Kagaku Foods Corporation, average particle size 610 µm, 850 g) and low-substituted hydroxypropylcellulose (50 g) were charged in a twin-screw kneader and processed at a kneading temperature of 108°C to give kneaded granules. The obtained granules were subjected to milling and screening in a screen mill (COMIL, screen hole diameter 2 mm grater-type). Microcrystalline cellulose (17.6 g), croscarmellose sodium (17.6 g), and light anhydrous silicic acid (3 g) were added to the obtained screened granules (300 g) and they were mixed. Magnesium stearate was sprayed (external lubricating method) and the mixture was tableted (tablet diameter 7 mmφ flat plane corner angle, compression pressure 1000 kgf) to give 150 mg tablets.

### Example 7

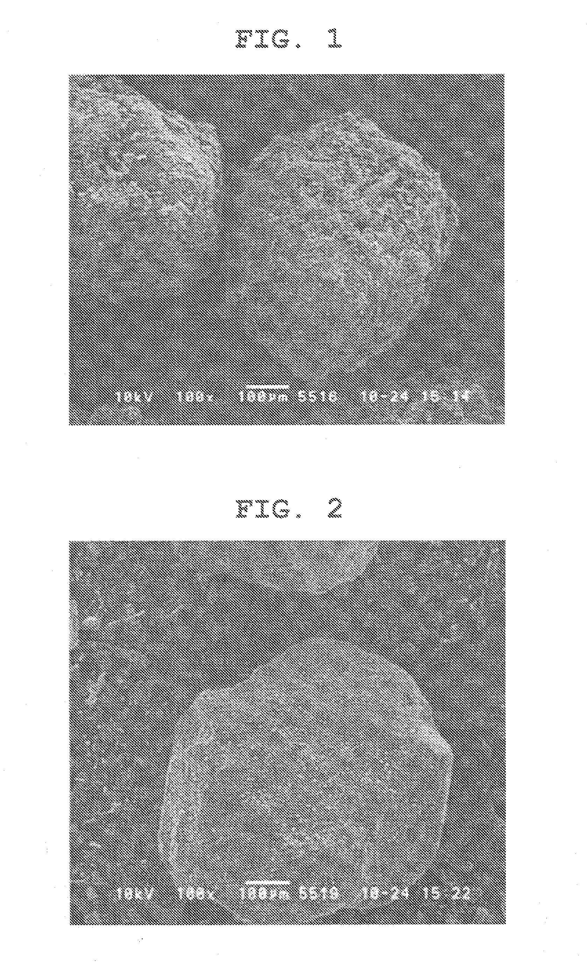

Erythritol (Mitsubishi-Kagaku Foods Corporation, average particle size 610 µm) was subjected to milling and screening in a screen mill (COMIL, screen hole diameter 0.81 mm), sieved with 500 µm and 150 µm sieves to give erythritol crystals having an average particle size of 430 µm. The obtained erythritol (500 g) having an average particle size of 430 µm was charged in a centrifugal rotary granulating-coating apparatus, and a mixture of microcrystalline cellulose (30 g), low-substituted hydroxypropylcellulose (30 g) and croscarmellose sodium (30 g) was added while spraying purified water (141 g), and the mixture was subjected to powder coating granulation, which was followed by a drying step to give granules (Fig. 1). Light anhydrous silicic acid (5.1 g) was added to the obtained granules and they were mixed. Magnesium stearate was sprayed (external lubricating method) and the mixture was tableted (tablet diameter 7 mmΦ flat plane corner angle, compression pressure 800 kgf) to give 150 mg tablets.

### Example 8

α-form mannitol (Pearlitol 200SD, Roquette Pharma, average particle size 200 µm, dissolution rate 4.62[1/min·m²], 530 g) was charged in a centrifugal rotary granulating-coating apparatus, and a mixture of microcrystalline cellulose (32 g) and low-substituted hydroxypropylcellulose (32 g) was added while spraying purified water (100 g), and the mixture was subjected to powder coating granulation, which was followed by a drying step to give granules. Light anhydrous silicic acid (1.7 g) was added to the obtained granules (199 g) and they were mixed. Magnesium stearate was sprayed (external lubricating method) and the mixture was tableted (tablet diameter 7 mmΦ flat plane corner angle, compression pressure 500 kgf) to give 150 mg tablets.

### Example 9

Lactose (SuperTab 11SD, DMV, average particle size 130 µm, dissolution rate 5.68[1/min·m²], 504 g) was charged in a centrifugal rotary granulating-coating apparatus, and a mixture of microcrystalline cellulose (30 g) and low-substituted hydroxypropylcellulose (30 g) was added while spraying purified water (110 g), and the mixture was subjected to powder coating granulation, which was followed by a drying step to give granules. Light anhydrous silicic acid (2 g) was added to the obtained granules (228 g) and they were mixed. Magnesium stearate was sprayed (external lubricating method) and the mixture was tableted (tablet diameter 7 mmΦ flat plane corner angle, compression pressure 500 kgf) to give 150 mg tablets.

### Example 10

Erythritol (Mitsubishi-Kagaku Foods Corporation, average particle size about 610 µm, 533 g) was charged in a centrifugal rotary granulating-coating apparatus, and a mixture of microcrystalline cellulose (32 g) and low-substituted hydroxypropylcellulose (32 g) was added while spraying purified water (160 g), and the mixture was subjected to powder coating granulation, which was followed by a drying step to give granules. Light anhydrous silicic acid (2 g) was added to the obtained granules (228 g) and they were mixed. Magnesium stearate was sprayed (external lubricating method) and the mixture was tableted (tablet diameter 7 mmΦ flat plane corner angle, compression pressure 500 kgf) to give 150 mg tablets.

### Example 11

Erythritol (Mitsubishi-Kagaku Foods Corporation, average particle size 610 µm) was subjected to milling and screening in a screen mill (COMIL, screen hole diameter 0.45 mm), sieved with an aperture 150 µm sieve to give erythritol crystals having an average particle size 100 µm. The obtained erythritol (504 g) having a particle size of 100 µm was charged in a centrifugal rotary granulating-coating apparatus, and a mixture of microcrystalline cellulose (30 g), low-substituted hydroxypropylcellulose (30 g) and croscarmellose sodium (30 g) was added while spraying purified water (100 g), and the mixture was subjected to powder coating granulation, which was followed by a drying step to give granules. Light anhydrous silicic acid (1.7 g) was added to the obtained granules (198 g). Magnesium stearate was sprayed (external lubricating method) and the mixture was tableted (tablet diameter 7 mmΦ flat plane corner angle, compression pressure 600 kgf) to give 120 mg tablets.

### Example 12

Erythritol (Mitsubishi-Kagaku Foods Corporation, average particle size about 610 µm) was milled in a hammer mill (IKA, screen size 2.0 mm, rotating speed 2000 min⁻¹), sieved with 250 µm and 150 µm sieves to give erythritol crystals having an average particle size 230 µm. The obtained erythritol (504 g) having an average particle size of 230 µm was charged in a centrifugal rotary granulating-coating apparatus, and a mixture of microcrystalline cellulose (30 g), low-substituted hydroxypropylcellulose (30 g) and croscarmellose sodium (30 g) was added while spraying purified water (100 g), and the mixture was subjected to powder coating granulation, which was followed by a drying step to give granules. Light anhydrous silicic acid (1.7 g) was added to the obtained granules (198 g). Magnesium stearate was sprayed (external lubricating method) and the mixture was tableted (tablet diameter 7 mmΦ flat plane corner angle, compression pressure 500 kgf) to give 150 mg tablets.

### Example 13

Erythritol(Mitsubishi-Kagaku Foods Corporation, average particle size about 610 µm) was milled in a hammer mill (screen size 2.0 mm, rotating speed 2000 min⁻¹), sieved with 355 µm and 250 µm sieves to give erythritol crystals having an average particle size of 330 µm. The obtained erythritol (531 g) having an average particle size of 330 µm was charged in a centrifugal rotary granulating-coating apparatus, and a mixture of microcrystalline cellulose (32 g) and low-substituted hydroxypropylcellulose (32 g) was added while spraying purified water (120 g), and the mixture was subjected to powder coating granulation, which was followed by a drying step to give granules. Light anhydrous silicic acid (1.7 g) was added to the obtained granules (198 g) and they were mixed. Magnesium stearate was sprayed (external lubricating method) and the mixture was tableted (tablet diameter 7 mmΦ flat plane corner angle, compression pressure 700 kgf, 1000 kgf) to give 150 mg tablets.

### Example 14

Erythritol (Mitsubishi-Kagaku Foods Corporation, average particle size about 610 µm) was milled in a hammer mill (screen size 2.0 mm, rotating speed 2000 min⁻¹), sieved with 250 µm and 150 µm sieves to give erythritol crystals having an average particle size of 230 µm. The obtained erythritol (521 g) having an average particle size of 230 µm was charged in a centrifugal rotary granulating-coating apparatus, and a mixture of microcrystalline cellulose (31 g) and low-substituted hydroxypropylcellulose (31 g) was added while spraying purified water (120 g), and the mixture was subjected to powder coating granulation, which was followed by a drying step to give granules. Light anhydrous silicic acid (1.7 g) was added to the obtained granules (198 g) and they were mixed. Magnesium stearate was sprayed (external lubricating method) and the mixture was tableted (tablet diameter 7 mmΦ flat plane corner angle, compression pressure 700 kgf, 1000 kgf) to give 150 mg tablets.

### Example 15

Erythritol (Mitsubishi-Kagaku Foods Corporation, average particle size 30 µm, 200 g) was charged in a high shear granulator (POWREX, VG-10), purified water (30 g) was added and the mixture was stirred for 5 min to give moistened granules. The granules were dried in a vacuum dryer for 1 hr, subjected to screening in a screen mill (COMIL, screen hole diameter 1.4 mm) and dried again in a vacuum dryer for 1 hr. The obtained dry granule was subjected to screening in a screen mill (COMIL, screen hole diameter 0.45 mm), and sieved with 355 µm and 250 µm sieves to give erythritol granules having an average particle size of 260 µm. The obtained erythritol granules (533 g) having an average particle size of 260 µm was charged in a centrifugal rotary granulating-coating apparatus, and a mixture of microcrystalline cellulose (31 g) and low-substituted hydroxypropylcellulose (31 g) was added while spraying purified water (100 g), and the mixture was subjected to powder coating granulation, which was followed by a drying step to give granules. Light anhydrous silicic acid (2.0 g) was added to the obtained granules (228 g) and they were mixed. Magnesium stearate was sprayed (external lubricating method) and the mixture was tableted (tablet diameter 7 mmΦ flat plane corner angle, compression pressure 500 kgf) to give 150 mg tablets.

### Example 16

Erythritol (Mitsubishi-Kagaku Foods Corporation, average particle size 30 µm, 200 g) was charged in a high shear granulator (POWREX, VG-10), purified water (30 g) was added and the mixture was stirred for 5 min to give moistened granules. The granules were dried in a vacuum dryer for 1 hr, subjected to screening in a screen mill (COMIL, screen hole diameter 1.4 mm) and dried again in a vacuum dryer for 1 hr. The obtained dry powder was subjected to screening in a screen mill (COMIL, screen hole diameter 0.45 mm), and sieved with 250 µm and 150 µm sieves to give erythritol granules having an average particle size of 160 µm. The obtained erythritol granules (531 g) having an average particle size of 160 µm were charged in a centrifugal rotary granulating-coating apparatus, and a mixture of microcrystalline cellulose (32 g) and low-substituted hydroxypropylcellulose (31 g) was added while spraying purified water (100 g), and the mixture was subjected to powder coating granulation, which was followed by a drying step to give granules. Light anhydrous silicic acid (2.0 g) was added to the obtained granules (228 g) and they were mixed. Magnesium stearate was sprayed (external lubricating method) and the mixture was tableted (tablet diameter 7 mmΦ flat plane corner angle, compression pressure 500 kgf) to give 150 mg tablets.

### Example 17

Erythritol (Mitsubishi-Kagaku Foods Corporation, average particle size about 610 µm, 531 g) was charged in a centrifugal rotary granulating-coating apparatus, and a mixture of microcrystalline cellulose (Asahi Kasei Corporation, 31 g) and low-substituted hydroxypropylcellulose (31 g) was added while spraying purified water (100 g), and the mixture was subjected to powder coating granulation, which was followed by a drying step to give granules. Light anhydrous silicic acid (1.7 g) and sodium stearyl fumarate (2 g) were added to the obtained granules (196 g) and they were mixed. The mixture was tableted (tablet diameter 7 mm flat plane corner angle, compression pressure 900 kgf) to give 150 mg tablets.

### Example 18

Erythritol (Mitsubishi-Kagaku Foods Corporation, average particle size about 610 µm) was milled in a hammer mill (screen size 2.0 mm, rotating speed 2000 min⁻¹), sieved with 355 µm and 250 µm sieves to give erythritol crystals having an average particle size of 330 µm. The obtained erythritol (530 g) having an average particle size of 330 µm was charged in a centrifugal rotary granulating-coating apparatus, and a mixture of microcrystalline cellulose (31 g) and low-substituted hydroxypropylcellulose (31 g) was added while spraying purified water (110 g), and the mixture was subjected to powder coating granulation, which was followed by a drying step to give granules. Magnesium stearate (1 g) was added to the obtained granules (199 g) and they were mixed. The mixture was tableted (tablet diameter 7 mmΦ flat plane corner angle, compression pressure 700 kgf) to give 150 mg tablets.

### Example 19

Erythritol (Mitsubishi-Kagaku Foods Corporation, average particle size about 610 µm) was milled in a hammer mill (screen size 2.0 mm, rotating speed 2000 min⁻¹), sieved with 355 µm and 250 µm sieves to give erythritol crystals having an average particle size of 330 µm. The obtained erythritol (530 g) having an average particle size of 330 µm was charged in a centrifugal rotary granulating-coating apparatus, and a mixture of microcrystalline cellulose (31 g) and low-substituted hydroxypropylcellulose (31 g) was added while spraying purified water (110 g), and the mixture was subjected to powder coating granulation, which was followed by a drying step to give granules. Magnesium stearate was sprayed (external lubricating method) and the obtained granules (200 g) was tableted (tablet diameter 7 mmΦ flat plane corner angle, compression pressure 700 kgf) to give 150 mg tablets.

### Example 20

Erythritol (Mitsubishi-Kagaku Foods Corporation, average particle size about 610 µm, 504 g) was charged in a centrifugal rotary granulating-coating apparatus, and a mixture of microcrystalline cellulose (30 g) and low-substituted hydroxypropylcellulose (30 g) was added while spraying purified water (100 g), and the mixture was subjected to powder coating granulation, which was followed by a drying step to give granules. Light anhydrous silicic acid (1.7 g) was added to the obtained granules (198 g). Magnesium stearate was sprayed (external lubricating method) and the mixture was tableted (tablet diameter 7 mmΦ flat plane corner angle, compression pressure 600 kgf) to give 120 mg tablets.

### Example 21

Erythritol (Mitsubishi-Kagaku Foods Corporation, average particle size about 610 µm, 465 g) was charged in a centrifugal rotary granulating-coating apparatus, and a mixture of microcrystalline cellulose (50 g) and low-substituted hydroxypropylcellulose (50 g) was added while spraying purified water (170 g), and the mixture was subjected to powder coating granulation, which was followed by a drying step to give granules. Light anhydrous silicic acid (2 g) was added to the obtained granules (228 g) and they were mixed. Magnesium stearate was sprayed (external lubricating method) and the mixture was tableted (tablet diameter 7 mmΦ flat plane corner angle, compression pressure 700 kgf) to give 150 mg tablets.

### Example 22

Erythritol (Mitsubishi-Kagaku Foods Corporation, average particle size about 610 µm, 532 g) was charged in a centrifugal rotary granulating-coating apparatus, and a mixture of microcrystalline cellulose (31 g) and low-substituted hydroxypropylcellulose (31 g) was added while spraying purified water (100 g), and the mixture was subjected to powder coating granulation, which was followed by a drying step to give granules. Ascorbic acid (13 g) and light anhydrous silicic acid (1.7 g) were added to the obtained granules (185 g) and they were mixed. Magnesium stearate was sprayed (external lubricating method) and the mixture was tableted (tablet diameter 7 mmΦ flat plane corner angle, compression pressure 900 kgf, 1200 kgf) to give 150 mg tablets.

### Example 23

Erythritol (Mitsubishi-Kagaku Foods Corporation, average particle size about 610 µm) was milled in a hammer mill (IKA, screen size 2.0 mm, rotating speed 2000 min⁻¹), sieved with 355 µm and 250 µm sieves to give erythritol crystals having an average particle size of 330 µm. The obtained erythritol (530 g) having an average particle size of 330 µm was charged in a centrifugal rotary granulating-coating apparatus, and a mixture of microcrystalline cellulose (31 g) and low-substituted hydroxypropylcellulose (31 g) was added while spraying purified water (110 g), and the mixture was subjected to powder coating granulation, which was followed by a drying step to give granules. Ascorbic acid (13 g) and light anhydrous silicic acid (1.7 g) were added to the obtained granules (185 g) and they were mixed. Magnesium stearate was sprayed (external lubricating method) and the mixture was tableted (tablet diameter 7 mmΦ flat plane corner angle, compression pressure 700 kgf) to give 150 mg tablets.

### Example 24

Erythritol (Mitsubishi-Kagaku Foods Corporation, average particle size about 610 µm, 532 g) was charged in a centrifugal rotary granulating-coating apparatus, and a mixture of microcrystalline cellulose (31 g) and low-substituted hydroxypropylcellulose (31 g) was added while spraying purified water (100 g), and the mixture was subjected to powder coating granulation, which was followed by a drying step to give granules A.
Then, acetaminophen (APIC, 80 g), microcrystalline cellulose (18 g) and hydroxypropylcellulose (2 g, Nippon Soda Co., Ltd.) were added into a powder particle processing machine (MECHANOMIL, OKADA-SEIKO CO. LTD.), water (18 g) was added and the mixture was subjected to stirring granulation. The obtained granules were dried in a ventilation dryer for 3 hr, and sieved with 350 µm and 250 µm sieves to give granules B having an average particle size of 300 µm.
Granules B (16 g) and light anhydrous silicic acid (1.7 g) was added to the obtained granules A (182 g) and they were mixed. Magnesium stearate was sprayed (external lubricating method) and the mixture was tableted (tablet diameter 7 mmΦ flat plane corner angle, compression pressure 700 kgf, 1000 kgf) to give 150 mg tablets.

### Example 25

Erythritol (Mitsubishi-Kagaku Foods Corporation, average particle size about 610 µm) was milled in a hammer mill (IKA, screen size 2.0 mm, rotating speed 2000 min⁻¹), sieved with 250 µm and 150 µm sieves to give erythritol crystals having an average particle size of 230 µm. The obtained erythritol (521 g) having an average particle size of 230 µm was charged in a centrifugal rotary granulating-coating apparatus, and a mixture of microcrystalline cellulose (31 g) and low-substituted hydroxypropylcellulose (31 g) was added while spraying purified water (100 g), and the mixture was subjected to powder coating granulation, which was followed by a drying step to give granules A. Then, acetaminophen (80 g), microcrystalline cellulose (18 g) and hydroxypropylcellulose (2 g) were added in to a powder particle processing machine, water (18 g) was added and the mixture was subjected to stirring granulation. The obtained granules were dried in a ventilation dryer for 3 hr, and sieved with 250 µm and 150 µm sieves to give granules B having an average particle size of 200 µm. Granules B (16 g) and light anhydrous silicic acid (1.7 g) were added to the obtained granules A (182 g) and they were mixed. Magnesium stearate was sprayed (external lubricating method) and the mixture was tableted (tablet diameter 7 mmΦ flat plane corner angle, compression pressure 400 kgf, 700 kgf) to give 150 mg tablets.

### Example 26

Erythritol (Mitsubishi-Kagaku Foods Corporation, average particle size 610 µm, 9.4 g) and low-substituted hydroxypropylcellulose (0.6 g) were charged in a twin-screw kneader (Kurimoto, Ltd., S1KRC kneader) and processed at a kneading temperature of 115°C to give kneaded granules. Microcrystalline cellulose (Asahi Kasei Corporation, 100 mg), croscarmellose sodium (100 mg), and light anhydrous silicic acid (17 mg) were added to the obtained granules (1.8 g) and they were mixed. Magnesium stearate was directly applied to the punch, and the mixture was tableted (KIKUSUI SEISAKUSHO LTD., correct 12HUK, tablet diameter 8 mmΦ flat plane corner angle, compression pressure 1000 kgf) to give 200 mg tablets.

### Example 27

Erythritol (Mitsubishi-Kagaku Foods Corporation, average particle size 610 µm, 9.4 g) and microcrystalline cellulose (0.6 g) were charged in a twin-screw kneader and processed at a kneading temperature of 115°C to give kneaded granules. Low-substituted hydroxypropylcellulose (100 mg), croscarmellose sodium (100 mg), and light anhydrous silicic acid (17 mg) were added to the obtained granules (1.8 g) and they were mixed. Magnesium stearate was directly applied to the punch, and the mixture was tableted (tablet diameter 8 mmΦ flat plane corner angle, compression pressure 1000 kgf) to give 200 mg tablets.

### Comparative Example 1

Erythritol (Mitsubishi-Kagaku Foods Corporation, average particle size 610 µm, 3.4 g), microcrystalline cellulose (200 mg), low-substituted hydroxypropylcellulose (200 mg), croscarmellose sodium (200 mg), and light anhydrous silicic acid (34 mg) were added and they were mixed. The mixture was tableted (tablet diameter 8 mmΦ flat plane corner angle, compression pressure 1000 kgf) to give a 200 mg tablet.

### Comparative Example 2

The additives in the same amount as in Example 3 were physically mixed to give a mixture. Magnesium stearate was sprayed (external lubricating method) and the obtained mixture was tableted (tablet diameter 7 mmΦ flat plane corner angle, compression pressure 1000 kgf) to give 150 mg tablets. The operation was the same as in Example 3 except that physical mixing was performed without using a centrifugal rotary granulating-coating apparatus.

### Comparative Example 3

Erythritol fine powder (Mitsubishi-Kagaku Foods Corporation, average particle size 31 µm, 842 g), microcrystalline cellulose (50 g), low-substituted hydroxypropylcellulose (50 g), and croscarmellose sodium (50 g) were charged in a rotary fluid bed granulator (POWREX CORPORATION, multiplex MP-01), and purified water (700 g) was sprayed to give granules. Light anhydrous silicic acid (8.5 g) was added to the obtained granules (960 g) and they were mixed Magnesium stearate was sprayed (external lubricating method) and the mixture was tableted (tablet diameter 7 mmΦ flat plane corner angle, compression pressure 600 kgf) to give 150 mg tablets.

### Comparative Example 4

The additives in the same amount as in Example 7 were physically mixed to give a mixture (Fig. 2). Magnesium stearate was sprayed (external lubricating method) and the obtained mixture was tableted (tablet diameter 7 mmΦ flat plane corner angle, compression pressure 800 kgf) to give 150 mg tablets. The operation was the same as in Example 7 except that physical mixing was performed without using a centrifugal rotary granulating-coating apparatus.

### Comparative Example 5

β-form mannitol (Parteck M300 Merck, average particle size 200 µm, dissolution rate 2.28[1/min·m²], 530 g) was charged in a centrifugal rotary granulating-coating apparatus, and a mixture of microcrystalline cellulose (32 g) and low-substituted hydroxypropylcellulose (32 g) was added while spraying purified water (190 g), and the mixture was subjected to powder coating granulation, which was followed by a drying step to give granules. Light anhydrous silicic acid (2 g) was added to the obtained granules (228 g) and they were mixed. Magnesium stearate was sprayed (external lubricating method) and the mixture was tableted (tablet diameter 7 mmΦ flat plane corner angle, compression pressure 300 kgf) to give 150 mg tablets.

### Comparative Example 6

Xylitol (Xylit, Mitsubishi Shoji Foodtech Co., Ltd., average particle size 500 µm, dissolution rate 3.57[1/min·m²], 504 g) was charged in a centrifugal rotary granulating-coating apparatus, and a mixture of microcrystalline cellulose (30 g) and low-substituted hydroxypropylcellulose (30 g) was added while spraying purified water (90 g), and the mixture was subjected to powder coating granulation, which was followed by a drying step to give granules. Light anhydrous silicic acid (2 g) was added to the obtained granules (228 g) and they were mixed. Magnesium stearate was sprayed (external lubricating method) and the mixture was tableted (tablet diameter 7 mmΦ flat plane corner angle, compression pressure 300 kgf) to give 150 mg tablets.

### Comparative Example 7

Purification sucrose (Wako Pure Chemical Industries, Ltd., average particle size 600 µm, dissolution rate 2.80[1/min·m²], 508 g) was charged in a centrifugal rotary granulating-coating apparatus, and a mixture of microcrystalline cellulose (31 g) and low-substituted hydroxypropylcellulose (31 g) was added while spraying purified water (90 g), and the mixture was subjected to powder coating granulation, which was followed by a drying step to give granules. Light anhydrous silicic acid (2 g) was added to the obtained granules (228 g) and they were mixed. Magnesium stearate was sprayed (external lubricating method) and the mixture was tableted (tablet diameter 7 mmΦ flat plane corner angle, compression pressure 300 kgf) to give 150 mg tablets.

### Comparative Example 8

The additives in the same amount as in Example 13 were physically mixed to give a mixture. Magnesium stearate was sprayed (external lubricating method) and the obtained mixture was tableted (tablet diameter 7 mmΦ flat plane corner angle, compression pressure 500 kgf) to give 150 mg tablets. The operation was the same as in Example 13 except that physical mixing was performed without using a centrifugal rotary granulating-coating apparatus.

### Comparative Example 9

Erythritol (Mitsubishi-Kagaku Foods Corporation, average particle size 610 µm, 504 g) was charged in a centrifugal rotary granulating-coating apparatus, and cornstarch (Roquette, 90 g) was added while spraying purified water (100 g), and the mixture was subjected to powder coating granulation, which was followed by a drying step to give granules. Light anhydrous silicic acid (1.7 g) was added to the obtained granules (198 g). Magnesium stearate was sprayed (external lubricating method) and the mixture was tableted (tablet diameter 7 mmΦ flat plane corner angle, compression pressure 1000 kgf) to give 120 mg tablets.

### Comparative Example 10

Erythritol (Mitsubishi-Kagaku Foods Corporation, average particle size 610 µm, 445 g) was charged in a centrifugal rotary granulating-coating apparatus, and a mixture of microcrystalline cellulose (50 g), low-substituted hydroxypropylcellulose (50 g), and croscarmellose sodium (50 g) was added while spraying purified water (210 g), and the mixture was subjected to powder coating granulation, which was followed by a drying step to give granules. Light anhydrous silicic acid (2 g) was added to the obtained granules (228 g) and they were mixed. Magnesium stearate was sprayed (external lubricating method) and the mixture was tableted (tablet diameter 7 mmΦ flat plane corner angle, compression pressure 1000 kgf) to give 150 mg tablets. The Comparative Example has hardness of the same level with the tablets obtained in Examples 1 - 25 of the present invention, though characteristically with high grittiness.

### Comparative Example 11

Erythritol (Mitsubishi-Kagaku Foods Corporation, average particle size 610 µm, 504 g) was charged in a centrifugal rotary granulating-coating apparatus, and a mixture of microcrystalline cellulose (10 g), low-substituted hydroxypropylcellulose (10 g) and croscarmellose sodium (10 g) was added while spraying purified water (100 g), and the mixture was subjected to powder coating granulation, which was followed by a drying step to give granules. Light anhydrous silicic acid (2 g) was added to the obtained granules (228 g) and they were mixed. Magnesium stearate was sprayed (external lubricating method) and the mixture was tableted (tablet diameter 7 mmΦ flat plane corner angle, compression pressure 1000 kgf) to give 150 mg tablets.

### Comparative Example 12

Erythritol (Mitsubishi-Kagaku Foods Corporation, average particle size 610 µm, 3.4 g), microcrystalline cellulose (200 mg), low-substituted hydroxypropylcellulose (200 mg), croscarmellose sodium (200 mg), and light anhydrous silicic acid (34 mg) were added and they were mixed. Magnesium stearate was directly applied to the punch, and the mixture was tableted (tablet diameter 8 mmΦ flat plane corner angle, compression pressure 1000 kgf) to give 200 mg tablets.

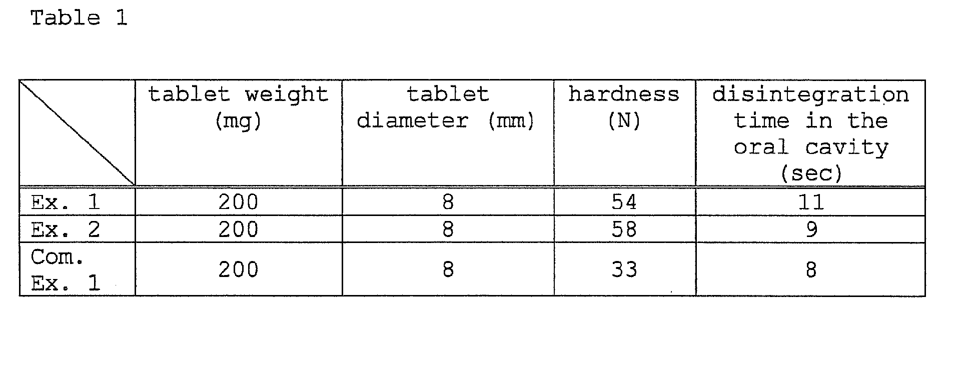

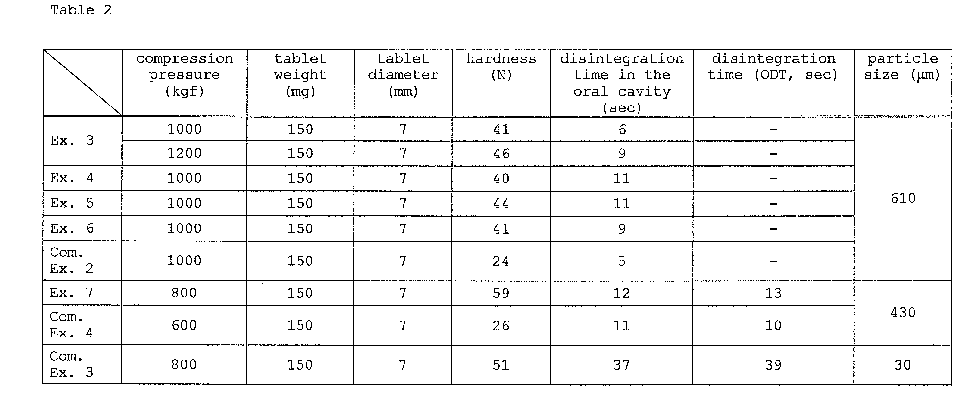

**Table 3**

| | tableting pressure (kgf) | tablet weight (mg) | tablet diameter (mm) | hard-ness (N) | disintegra-tion time (ODT, sec) | particle size (µm) |
|---|---|---|---|---|---|---|
| Ex. 11 | 600 | 120 | 7 | 49 | 19 | 100 |
| Ex. 12 | 500 | 150 | 7 | 55 | 15 | 230 |
| Ex. 13 | 700 | 150 | 7 | 52 | 8 | 330 |
| | 1000 | 150 | 7 | 64 | 10 | |
| Ex. 14 | 700 | 150 | 7 | 69 | 8 | 230 |
| | 1000 | 150 | 7 | 63 | 10 | |
| Ex. 15 | 500 | 150 | 7 | 57 | 13 | 155 |
| Ex. 16 | 500 | 150 | 7 | 51 | 13 | 255 |
| Com. Ex. 8 | 500 | 150 | 7 | 27 | 9 | 330 |

**Table 4**

| | tableting pressure (kgf) | tablet weight (mg) | tablet diameter (mm) | hard-ness (N) | disintegra-tion time (ODT, sec) | particle size (µm) |
|---|---|---|---|---|---|---|
| Com. Ex. 5 | 300 | 150 | 7 | 33 | 60 | 200 |
| Ex. 8 | 500 | 150 | 7 | 44 | 25 | 200 |
| Com. Ex. 6 | 300 | 150 | 7 | 48 | 37 | 530 |
| Com. Ex. 7 | 300 | 150 | 7 | 18 | 57 | 630 |
| Ex. 9 | 500 | 150 | 7 | 50 | 21 | 130 |
| Ex. 10 | 500 | 150 | 7 | 41 | 15 | 610 |

**Table 5**

| | tableting pressure (kgf) | tablet weight (mg) | tablet diameter (mm) | hard-ness (N) | disintegra-tion time (ODT, sec) | particle size (µm) |
|---|---|---|---|---|---|---|
| Ex. 10 | 500 | 150 | 7 | 41 | 15 | 610 |
| Ex. 17 | 900 | 150 | 7 | 45 | 15 | |
| Ex. 18 | 700 | 150 | 7 | 47 | 9 | 330 |
| Ex. 19 | 700 | 150 | 7 | 64 | 9 | |

**Table 6**

| | tablet weight (mg) | tablet diameter (mm) | hardness (N) | disintegration time (ODT, sec) | particle size (µm) |
|---|---|---|---|---|---|
| Ex. 20 | 120 | 7 | 42 | 12 | 610 |
| Ex. 10 | 120 | 7 | 49 | 19 | 100 |
| Com. Ex. 9 | 120 | 7 | 25 | - | 610 |
| Ex. 21 | 150 | 7 | 52 | 11 | |
| Com. Ex. 10 | 150 | 7 | 49 | 34 | |
| Com. Ex. 11 | 150 | 7 | 23 | - | |

**Table 7**

| Lot | tableting pressure (kgf) | tablet weight (mg) | hardness (N) | disintegration time (ODT, sec) | particle size (µm) |
|---|---|---|---|---|---|
| Ex. 22 | 900 | 150 | 44 | 11 | 610 |
| | 1200 | 150 | 43 | 15 | |
| Ex. 23 | 700 | 150 | 42 | 10 | 330 |
| Ex. 24 | 700 | 150 | 47 | 13 | 610 |
| | 1000 | 150 | 60 | 12 | |
| Ex. 25 | 400 | 150 | 45 | 12 | 230 |
| | 700 | 150 | 67 | 13 | |

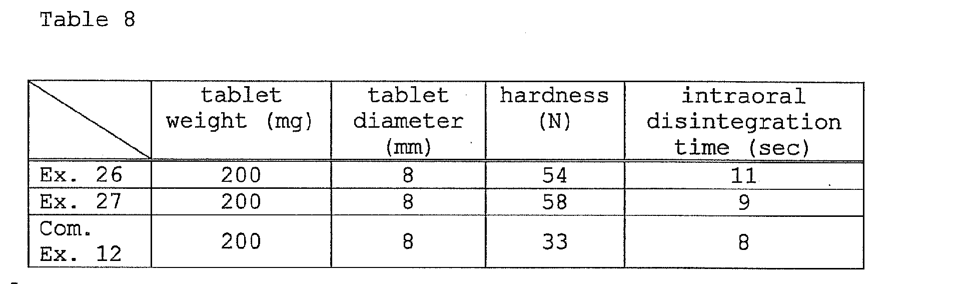

As shown in the above-mentioned Table 1, Table 2 and Table 3, the tablets obtained in Comparative Example 1, Comparative Example 2, Comparative Example 4, and Comparative Example 8 shows rapid disintegration in the oral cavity, but failed to afford satisfactory hardness. As shown in Table 2, moreover, the tablet obtained in Comparative Example 3 afforded satisfactory hardness, but disintegration time in the oral cavity was drastically delayed. As shown in Table 6, moreover, to achieve rapid disintegration and tablet strength sufficient for handling in Comparative Example 10 and Comparative Example 11, cellulose to be added by coating or added has an optimal value, which was clarified to be preferably 8 - 30 parts by weight relative to 100 parts by weight of saccharide or sugar alcohol to be the core.
As shown in Table 4, saccharide and sugar alcohol shown in Comparative Examples 5 - 7 failed to provide a preparation showing rapid disintegration in the oral cavity. It has been clarified that a dissolution rate of saccharide or sugar alcohol to be used as a core contributes to rapid disintegration. As shown in Comparative Example 9, when cornstarch was added as a coating additive onto the surface, sufficient hardness was not achieved. In contrast, the tablets obtained in Examples 1 - 25 of the present invention showed a disintegration time in the oral cavity similar to that in Comparative Examples 1, 2, 4 and 8, and satisfactory hardness.

### Industrial Applicability

According to the present invention, a rapidly disintegrating solid preparation such as tablet and the like can be provided, which has sufficient hardness that prevents damage during distribution process, and rapidly disintegrates in the oral cavity. As a result, it is easy to ingest even for elderly people and children having weak swallowing ability, and can be ingested even when water is not obtainable. According to the production method of the present invention, moreover, an orally rapidly disintegrating tablet can be produced conveniently at a low cost.

This application is based on a patent application No. 2007-250920 filed in Japan (filing date: September 27, 2007), the contents of which are incorporated in full herein by this reference.

## Claims

1. A rapidly disintegrating solid preparation comprising coated granules wherein saccharide or sugar alcohol having an average particle size of not less than 75 µm and a high dissolution rate is coated with cellulose.

2. The solid preparation according to claim 1, wherein said saccharide or sugar alcohol is a crystal particle or a granulated particle.

3. The solid preparation according to claim 2, wherein said saccharide or sugar alcohol is a crystal particle.

4. The solid preparation according to any one of claims 1 to 3, wherein said saccharide or sugar alcohol has an average particle size of not less than 100 µm.

5. The solid preparation according to claim 4, wherein said saccharide or sugar alcohol has an average particle size of 100 µm - 700 µm.

6. The solid preparation according to claim 5, wherein said saccharide or sugar alcohol has an average particle size of 100 µm - 400 µm.

7. The solid preparation according to any of claims 1 to 5, wherein said saccharide or sugar alcohol has an average particle size of not less than 400 µm.

8. The solid preparation according to any one of claims 1 to 7, wherein said saccharide or sugar alcohol has a dissolution rate of not less than 4.0 [1/min·m²].

9. The solid preparation according to claim 8, wherein said saccharide or sugar alcohol has a dissolution rate of not less than 4.5 [1/min·m²].

10. The solid preparation according to any one of claims 1 to 9, wherein said saccharide or sugar alcohol is one or more kinds selected from erythritol, α-form mannitol and lactose.

11. The solid preparation according to claim 10, wherein said saccharide or sugar alcohol is erythritol.

12. The solid preparation according to any one of claims 1 to 11, having a hardness of not less than 35N and a disintegration time of 30 seconds or less.

13. The solid preparation according to claim 12, wherein said hardness is not less than 35N and said disintegration time is 15 seconds or less.

14. The solid preparation according to any one of claims 1 to 13, wherein said cellulose for coating is 8 - 30 parts by weight relative to 100 parts by weight of said saccharide or sugar alcohol.

15. The solid preparation according to claim 14, wherein said cellulose for coating is 10 - 20 parts by weight relative to 100 parts by weight of said saccharide or sugar alcohol.

16. A rapidly disintegrating solid preparation comprising a) an active ingredient, b) saccharide or sugar alcohol having an average particle size of not less than 400 µm, c) cellulose and d) a disintegrant.
